# EUROPÄISCHE PATENTSCHRIFT

(11) **EP 0 751 122 B1**
(45) Veröffentlichungstag und Bekanntmachung des Hinweises auf die Patenterteilung: **01.12.1999**
(21) Anmeldenummer: 96110316.5
(22) Anmeldetag: 26.06.1996
(51) Int. Cl.: C07C 273/18, C07C 275/70

(54) **Verfahren zur Herstellung von kristallinem O-Isopropylisoharnstoff-hydrochlorid**
Process for the preparation of crystalline O-isopropyl isourea-hydrochloride
Procédé de préparation d'O-isopropyl isourée hydrochlorure cristalline

(30) Priorität: 27.06.1995 DE 19523205
(43) Veröffentlichungstag der Anmeldung: 02.01.1997
(73) Patentinhaber: SKW Trostberg Aktiengesellschaft, 83308 Trostberg (DE)
(72) Erfinder: Weiss, Stefan, Dr., 83308 Trostberg (DE)
(74) Vertreter: Weiss, Wolfgang, Dipl.-Chem. Dr.

(56) Entgegenhaltungen:
- DE-A- 1 948 370
- US-A- 3 551 489

## Beschreibung

Die vorliegende Erfindung betrifft ein Verfahren zur Herstellung von kristallinem O-Isopropylisoharnstoff-hydrochlorid mit hoher Reinheit, welches ein wichtiges Zwischenprodukt für die Herstellung von Pflanzenschutz- und Arzneimitteln darstellt.

Die Herstellung dieser Verbindung ist mehrfach beschrieben, doch war es bisher nicht möglich, reines O-Isopropylisoharnstoff-hydrochlorid in kristalliner Form zu gewinnen. So beschreiben Basterfield und Powell die Umsetzung von Cyanamid mit Chlorwasserstoff in Isopropanol (vgl. Canad.J.Res. 1, 261 (1929)). Hierbei fällt das Produkt in Form eines gelben, viskosen Öls an, welches auch aus einer etherischen Lösung bei -10°C nicht auskristallisiert werden kann.

Entsprechend den Patenten US 3,551,489 und GB 1,194,313 kann man O-Isopropylisoharnstoff-hydrochlorid durch Reaktion von Cyanamid mit konzentrierter Salzsäure und Isopropanol in Form eines Sirups gewinnen, aus dem zwar O-Isopropylisoharnstoffpikrat, nicht aber das Hydrochlorid selbst in kristalliner Form isoliert werden kann.

Entsprechend dem Stand der Technik ist also die Herstellung von kristallinem und reinem 0-Isopropylharnstoff-hydrochlorid durch Umsetzung von Cyanamid mit Isopropanol in Gegenwart von Chlorwasserstoff oder Salzsäure nicht möglich, da bei dieser Reaktion nur eine unreine Verbindung in Form eines Öls oder Sirups gewonnen werden kann. Die Handhabung des daraus isolierten Pikrats ist im technischen Maßstab gefährlich.

Außerdem ist eine Reinigung der Substanz durch Umkristallisation nicht möglich und die Verwendung unreiner Substanzen bei der Synthese wertvoller Wirkstoffe, z.B. in der Pharmasynthese, oftmals nicht möglich oder mit großen Problemen verbunden.

Ein weiterer Nachteil der beschriebenen Verfahren besteht darin, daß mit gasförmigem Chlorwasserstoff oder mit konzentrierter Salzsäure gearbeitet werden muß, welche metallische Werkstoffe, wie Edelstahl, stark angreifen und durch Korrosion zerstören.

Schließlich wird auch in der DE-OS 19 48 370 die Herstellung von O-Isopropylisoharnstoff-hydrochlorid durch Erhitzen von Chlorformamidinium-chlorid in Isopropanol beschrieben. Nach längerem Stehen im Kühlschrank kann hierbei ein festes Produkt vom Schmelzpunkt 57 bis 61°C gewonnen werden, das jedoch noch mit Chlorformamidinium-chlorid verunreinigt ist.

Auch dieses Verfahren ist für die Herstellung von kristallinem und reinem O-Isopropylisoharnstoff-hydrochlorid im technischen Maßstab nicht geeignet, da eine längere Lagerung bei tiefen Temperaturen bei einer technischen Herstellung sehr aufwendig ist.

Außerdem wird bei der Reaktion von Chlorformamidinium- chlorid mit Isopropanol Chlorwasserstoff in äquimolaren Mengen freigesetzt, der sich nur langsam und nicht vollständig zu Isopropylchlorid umsetzt. Daher enthält auch dieses Reaktionsgemisch freien Chlorwasserstoff, der sehr korrosiv ist.

Des weiteren entsteht als unerwünschtes Nebenprodukt das leicht flüchtige Isopropylchlorid. Die Abtrennung und Vernichtung dieses Chlorkohlenwasserstoffes ist sehr aufwendig und kostspielig, da er nicht in die Umwelt gelangen darf. Isopropylchlorid gehört zudem zu den Alkylierungsmitteln, die bekanntermaßen mutagene Eigenschaften aufweisen.

Schließlich enthält das auf diese Weise hergestellte O-Isopropylisoharnstoff-hydrochlorid Harnstoff als Verunreinigung, da O-Alkylisoharnstoff-hydrochloride beim Erhitzen bekanntermaßen in Harnstoff und Alkylhalogenide zerfallen.

Der vorliegenden Erfindung lag daher die Aufgabe zugrunde, ein Verfahren zur Herstellung von O-Isopropylisoharnstoffhydrochlorid zu entwickeln, welches die genannten Nachteile des Standes der Technik nicht aufweist, sondern mit geringem technischen Aufwand und in umweltschonender Weise die Herstellung eines kristallinen Produkts mit hoher Reinheit ermöglicht.

Diese Aufgabe wurde erfindungsgemäß dadurch gelöst, daß man Cyanamid, Chlorformamidinium-chlorid und Isopropanol umsetzt, dem Reaktionsgemisch vor, während oder/und nach der Umsetzung ein aprotisches organisches Lösemittel ausgewählt aus der Gruppe der Ketone, Ether, Ester oder/und Acetale zusetzt und ggf. nach Abkühlung der Reaktionslösung das kristalline Reaktionsprodukt abtrennt.

Es hat sich nämlich überraschenderweise gezeigt, daß man auf diese Weise das Reaktionsprodukt in einer sehr reinen und grobkristallinen Form gewinnen kann, welches leicht von der Reaktionslösung abgetrennt werden kann.

Beim Verfahren entsprechend der vorliegenden Erfindung werden also Cyanamid, Chlorformamidinium-chlorid und Isopropanol zur Reaktion gebracht. Das Molverhältnis Cyanamid zu Chlorformamidinium-chlorid kann hierbei in relativ weiten Grenzen variiert werden, wobei vorzugsweise ein merklicher Überschuß oder Unterschuß von Chlorformamidinium-chlorid -chlorid vermieden wird. Vorzugsweise wird ein Reaktionsgemisch entsprechend einem Molverhältnis Cyanamid zu Chlorformamidinium-chlorid von 1 : 0,5 bis 1 : 2 verwendet.

Gemäß einer besonders bevorzugten Ausführungsform wird ein äquimolares oder annähernd äquimolares Gemisch von Cyanamid und Chlorformamidinium-chlorid eingesetzt. Das Verhältnis Cyanamid zu Isopropanol kann ebenfalls in weiten Grenzen variiert werden, doch hat es sich als besonders vorteilhaft erwiesen, pro Mol Cyanamid 2 bis 10 Mol, vorzugsweise 2,5 bis 3,5 Mol, Isopropanol zu verwenden.

Gemäß einer bevorzugten Ausführungsform kann man das zur Herstellung benötigte Chlorformamidinium-chlorid ohne nachfolgende Isolierung aus Chlorwasserstoff und Cyanamid in Isopropanol, ggf. in Anwesenheit eines weiteren Lösemittels, z.B. eines Ethers oder Esters, vorzugsweise Isopropylacetat, erzeugen.

Ebenso ist es möglich, das Reaktionsgemisch aus Cyanamid und Chlorformamidinium-chlorid in-situ durch Umsetzung von überschüssigem Cyanamid und Chlorwasserstoff in Isopropanol ebenfalls evtl. in Anwesenheit eines weiteren Lösemittels herzustellen.

Es ist als erfindungswesentlich anzusehen, daß man dem Reaktionsgemisch vor, während oder/und nach der Umsetzung ein aprotisches organisches Lösemittel zusetzt, um so das Reaktionsgemisch zu verdünnen. Als organisches Lösemittel wird hierbei ein aprotisches Solvens aus der Gruppe der Ketone, Ether, Ester oder/und Acetale herangezogen, und zwar besonders bevorzugt in einer solchen Menge, daß auf 1 Mol Cyanamid 50 bis 1000 g, insbesondere 300 bis 500 g, organisches Lösemittel kommen.

Als bevorzugte organische Lösemittel finden hierbei Aceton oder Isopropylacetat Verwendung. Natürlich kann noch auf weitere Ether, z.B. Diethylether oder Tetrahydrofuran, sowie Ester, z.B. Ethylacetat, oder Acetale, wie Acetaldehyddimethylacetal, zurückgegriffen werden. Die Durchführung der Reaktion, die vorzugsweise zwischen 0 und 100°C und besonders bevorzugt bei 30 bis 60°C, vorgenommen wird, ist relativ unkritisch, d.h. man kann beispielsweise Cyanamid und Isopropanol vorlegen und Chlorformamidinium-chlorid zugeben. Stattdessen kann man auch ohne weiteres das Isopropanol vorlegen und Cyanamid und Chlorformamidinium-chlorid nacheinander oder gleichzeitig zugeben.

Nach Durchführung der Reaktion bzw. Zugabe des organischen Lösemittels wird insbesondere dann, wenn bei relativ hohen Temperaturen gearbeitet wurde, vorzugsweise auf 10 bis 15°C abgekühlt und das dabei entstehende kristalline Reaktionsprodukt nach bekannten Methoden, insbesondere durch Filtration, abgetrennt. Durch Animpfen mit O-Isopropylisoharnstoff-hydrochlorid kann die Kristallisation beschleunigt werden.

Auf diese Weise ist die Herstellung von O-Isopropylisoharnstoff-hydrochlorid in technisch einfacher Weise aus den wohlfeilen Ausgangsstoffen Cyanamid und Chlorformamidinium-chlorid möglich, wobei es nicht erforderlich ist, die Reaktionslösung einzuengen und/oder anschließend längere Zeit bei tiefen Temperaturen zu lagern.

Durch das erfindungsgemäße Verfahren ist außerdem ein O-Isopropylisoharnstoff-hydrochlorid in hoher Reinheit erhältlich, welches einen deutlich höheren Schmelzpunkt als das aus dem Stand der Technik bekannte Produkt aufweist. Vorzugsweise ist der Schmelzpunkt mindestens 80°C, besonders bevorzugt mindestens 90°C und am meisten bevorzugt im Bereich von 95 bis 100°C.

Ein weiterer Vorteil des erfindungsgemäßen Verfahrens ist, daß weder Chlorwasserstoff oder Isopropylchlorid als Nebenprodukte freigesetzt werden. Außerdem wird beim erfindungsgemäßen Verfahren das gesamte Chlor in Chlorformamidinium-chlorid ausgenutzt, während entsprechend dem Stand der Technik die Hälfte des Chlorgehalts in Isopropylchlorid als unerwünschtes Nebenprodukt überführt wird.

Aufgrund dieser besonderen Vorteile ist das erfindungsgemäße Verfahren besonders gut für den technischen Maßstab geeignet.

Die nachfolgenden Beispiele sollen die Erfindung näher erläutern.

### Beispiele

### Beispiel 1

Zu einer Lösung von 126,6 g (3,0 Mol) 99,6 %igem kristallinem Cyanamid (SKW Cyanamid F 1000) in 625 g Isopropanol wurden unter Rühren 345 g (3,0 Mol) Chlorformamidinium-chlorid (SKW Trostberg AG) bei 20°C zugegeben. Anschließend wurde das Reaktionsgemisch auf 30°C erwärmt und bei dieser Temperatur solange gerührt, bis kein Cyanamid mehr im Reaktionsgemisch nachgewiesen werden konnte. (Der Cyanamidnachweis erfolgte mit ammoniakalischer Silbernitratlösung). Die Reaktionszeit betrug 130 Stunden. Dann wurden 1245 g Aceton zugesetzt. Es wurde noch 6 Stunden bei 15°C gerührt. Der kristalline Niederschlag wurde abgesaugt, mit 190 g Aceton gewaschen und im Vakuumtrockenschrank bei 50°C und 20 mbar getrocknet. Die Ausbeute betrug 518 g (3,74 Mol) o-Isopropylisoharnstoff-hydrochlorid vom Schmelzpunkt 98 - 99°C (Kapillare im Schmelzblock). Der Literaturschmelzpunkt beträgt 57 - 61°C (DE 19 48 370).

Eine Elementaranalyse des Produkts erbrachte die folgenden Resultate (Angaben in Prozent):

| | |
|---|---|
| C Ber. 34,66 | Gef. 34,56 |
| H Ber. 8,00 | Gef. 7,97 |
| Cl Ber. 25,58 | Gef. 25,60 |
| N Ber. 20,21 | Gef. 20,34 |
| Harnstoff: | < 0.10 % |

### Beispiel 2

253,5 g (6,0 Mol) 99,6 %iges kristallines Cyanamid (SKW Cyanamid F 1000) wurden in einer Mischung von 650 g Isopropanol und 1500 g Isopropylacetat gelöst. In diese Lösung wurden unter Rühren und äußerer Kühlung 218,8 g (6,0 Mol) Chlorwasserstoff in dem Maße eingeleitet, daß die Innentemperatur nicht über 15°C anstieg.

Anschließend wurde das aus 126,1 (3,0 Mol) Cyanamid und 345 g (3,0 Mol) Chlorformamidinium-chlorid bestehende Reaktionsgemisch auf 50°C erhitzt und bei dieser Temperatur 16 Stunden gerührt. Danach wurde auf 15°C abgekühlt und bei dieser Temperatur noch 6 Stunden nachgerührt. Der kristalline Rückstand wurde abgesaugt, mit Aceton gewaschen und im Vakuum bei 15 mbar getrocknet. Die Ausbeute betrug 682 g (49,2 %) reines O-Isopropylharnstoff-hydrochlorid vom Schmp. 98°C.

### Beispiel 3

160 g Isopropanol wurden vorgelegt und auf 60°C erhitzt. Bei dieser Temperatur wurden 42,1 g (1,0 Mol) 99,8 %iges kristallines Cyanamid (SKW Cyanamid F 1000) und 115 g (1,0 Mol) Chlorformamidinium-chlorid portionsweise in äquimolaren Mengen während 4 Stunden unter gutem Rühren zugegeben. Danach wurde noch 6 Stunden bei 60°C gerührt. Nach Zugabe von 300 g Aceton wurde auf 14°C abgekühlt und unter Wasserkühlung noch 4 Stunden ausgerührt. Das kristalline Reaktionsprodukt wurde abgesaugt, mit 80 g Aceton gewaschen und im Vakuumtrockenschrank bei 50°C/15 mbar getrocknet.

Ausbeute: 224 g (1,62 Mol) O-Isopropylisoharnstoff-hydrochlorid; Schmp. 97 - 99°C.

## Patentansprüche

1. Verfahren zur Herstellung von kristallinem O-Isopropylisoharnstoff-hydrochlorid,
**dadurch gekennzeichnet,**
daß man Cyanamid, Chlorfomamidinium-chlorid und Isopropanol umsetzt, dem Reaktionsgemisch vor, während oder/und nach der Umsetzung ein aprotisches organisches Lösemittel ausgewählt aus der Gruppe der Ketone, Ether, Ester oder/und Acetale zusetzt und ggf. nach Abkühlung der Reaktionslösung das kristalline Reaktionsprodukt abtrennt.

2. Verfahren nach Anspruch 1,
**dadurch gekennzeichnet**,
daß die Umsetzung bei Temperaturen von 0 bis 100°C erfolgt.

3. Verfahren nach Anspruch 1 oder 2,
**dadurch gekennzeichnet**,
daß das Molverhältnis Cyanamid zu Chlorformamidinium-chlorid 1 : 0,5 bis 1 : 2 beträgt.

4. Verfahren nach einem der Ansprüche 1 bis 3,
**dadurch gekennzeichnet**,
daß man pro Mol Cyanamid 2 bis 10 Mol, vorzugsweise 2,5 bis 3,5 Mol, Isopropanol einsetzt.

5. Verfahren nach einem der Ansprüche 1 bis 4,
**dadurch gekennzeichnet**,
daß man das organische Lösemittel in einer Menge von 50 bis 1000 g, insbesondere 300 bis 500 g, pro Mol Cyanamid verwendet.

6. Verfahren nach einem der Ansprüche 1 bis 5,
**dadurch gekennzeichnet**,
daß man als organisches Lösemittel Aceton einsetzt.

7. Verfahren nach einem der Ansprüche 1 bis 6,
**dadurch gekennzeichnet**,
daß man als organisches Lösemittel Isopropylacetat verwendet.

8. Verfahren nach einem der Ansprüche 1 bis 7,
**dadurch gekennzeichnet**,
daß man das Chlorformamidinium-chlorid ohne nachfolgende Isolierung aus Chlorwasserstoff und Cyanamid im Isopropanol ggf. in Anwesenheit eines weiteren Lösemittels erzeugt.

9. Verfahren nach einem der Ansprüche 1 bis 7,
**dadurch gekennzeichnet**,
daß ein Gemisch aus Cyanamid und Chlorformamidinium-chlorid in situ durch Umsetzung von Cyanamid und Chlorwasserstoff in Isopropanol ggf. in Anwesenheit eines weiteren Lösemittels hergestellt wird.

10. Verfahren nach einem der Ansprüche 1 bis 9,
**dadurch gekennzeichnet**,
daß man die Umsetzung bei Temperaturen von 30 bis 60°C durchführt.

11. Verfahren nach einem der Ansprüche 1 bis 10,
**dadurch gekennzeichnet**,
da man das Reaktionsgemisch nach der Umsetzung auf Temperaturen von 10 bis 15°C abkühlt.

12. Verfahren nach Anspruch 11,
**dadurch gekennzeichnet**,
daß man das Reaktionsgemisch durch Zugabe von kristallinem O-Isopropylisoharnstoff-hydrochloridd animpft.

## Claims

1. A method of preparing crystalline O-isopropylisourea hydrochloride, characterised in that cyanamide, chloroformamidinium chloride and isopropanol are reacted, before, during and/or after the reaction an aprotic organic solvent, selected from the group of the ketones, ethers, esters and/or acetals, is added to the reaction mixture and, optionally after cooling the reaction solution, the crystalline reaction product is separated.

2. A method according to Claim 1, characterised in that the reaction takes place at temperatures of 0 to 100°C.

3. A method according to Claim 1 or 2, characterised in that the molar ratio of cyanamide to chloroformamidinium chloride is 1 : 0.5 to 1 : 2.

4. A method according to any one of Claims 1 to 3, characterised in that 2 to 10 mol, preferably 2.5 to 3.5 mol, of isopropanol are used per mol of cyanamide.

5. A method according to any one of Claims 1 to 4, characterised in that the organic solvent is used in a quantity of 50 to 1000 g, particularly 300 to 500 g, per mol of cyanamide.

6. A method according to any one of Claims 1 to 5, characterised in that acetone is used as the organic solvent.

7. A method according to any one of Claims 1 to 6, characterised in that isopropyl acetate is used as the organic solvent.

8. A method according to any one of Claims 1 to 7, characterised in that the chloroformamidinium chloride is produced without subsequent isolation from hydrogen chloride and cyanamide in the isopropanol, optionally in the presence of a further solvent.

9. A method according to any one of Claims 1 to 7, characterised in that a mixture of cyanamide and chloroformamidinium chloride is prepared in situ by reacting cyanamide and hydrogen chloride in isopropanol, optionally in the presence of a further solvent.

10. A method according to any one of Claims 1 to 9, characterised in that the reaction is carried out at temperatures of 30 to 60°C.

11. A method according to any one of Claims 1 to 10, characterised in that, after the reaction, the reaction mixture is cooled to temperatures of 10 to 15°C.

12. A method according to Claim 11, characterised in that the reaction mixture is seeded by adding crystalline O-isopropyliso-urea hydrochloride.

## Revendications

1. Procédé de préparation de chlorhydrate de O-isopropyl iso-urée cristallin,
caractérisé en ce
que l'on transforme du cyanamide, du chlorure de chloroformamidine et d'isopropanol, et qu'avant, pendant et/ou après la transformation, on ajoute au mélange réactionnel un solvant organique aprotique choisi dans le groupe des cétone, ester et/ou acétal et qu'on sépare le produit de réaction cristallin, éventuellement après refroidissement de la solution réactionnelle.

2. Procédé selon la revendication 1, caractérisé en ce
que la transformation s'effectue à des températures de 0 à 100°C.

3. Procédé selon la revendication 1 ou 2,
caractérisé en ce
que le rapport molaire du cyanamide au chlorure de chloroformamidine est compris entre 1:0,5 et 1:2.

4. Procédé selon l'une des revendications 1 à 3,
caractérisé en ce
qu'on utilise 2 à 10 moles, de préférence 2,5 à 3,5 moles, d'isopropanol pour chaque mole de cyanamide.

5. Procédé selon l'une des revendications 1 à 4,
caractérisé en ce
qu'on utilise le solvant organique en une quantité de 50 à 1000 g, et notamment de 300 à 500 g, pour chaque mole de cyanamide.

6. Procédé selon l'une des revendications 1 à 5,
caractérisé en ce
qu'on utilise de l'acétone comme solvant organique.

7. Procédé selon l'une des revendications 1 à 6,
caractérisé en ce
qu'on utilise de l'acétate d'isopropyle comme solvant organique.

8. Procédé selon l'une des revendications 1 à 7,
caractérisé en ce
que l'on produit le chlorure de chloroformamidine sans isolation ultérieure à partir de gaz chlorhydrique et de cyanamide dans l'isopropanol, éventuellement en présence d'un autre solvant.

9. Procédé selon l'une des revendications 1 à 8,
caractérisé en ce
qu'on réalise un mélange formé de cyanamide et de chlorure de formamidine in situ par transformation de cyanamide et de gaz chlorhydrique dans l'isopropanol, éventuellement en présence d'un autre solvant.

10. Procédé selon l'une des revendications 1 à 9,
caractérisé en ce
qu'on exécute la transformation à des températures de 30 à 60°C.

11. Procédé selon l'une des revendications 1 à 10,
caractérisé en ce
qu'on refroidit le mélange réactionnel après la transformation, à une température de 10 à 15°C.

12. Procédé selon la revendication 11, caractérisé en ce qu'on amorce le mélange réactionnel par addition de chlorhydrate de O-isopropyl iso-urée cristallin.
